(19) Europäisches Patentamt
European Patent Office
Office européen des brevets

(11) **EP 2 178 384 B1**

(12) **EUROPEAN PATENT SPECIFICATION**

(45) Date of publication and mention
of the grant of the patent:
**20.06.2012   Bulletin 2012/25**

(21) Application number: **08774413.2**

(22) Date of filing: **27.06.2008**

(51) Int Cl.:
*A23C 9/137* *(2006.01)*      *A23C 9/133* *(2006.01)*

(86) International application number:
**PCT/EP2008/058246**

(87) International publication number:
**WO 2009/003931 (08.01.2009 Gazette 2009/02)**

(54) **NOVEL FUNCTIONAL FOOD PRODUCT CONTAINING A SPECIFIC FIBRE MIXTURE**

NEUES FUNKTIONELLES LEBENSMITTELPRODUKT MIT EINEM SPEZIFISCHEN FASERGEMISCH

NOUVEAU PRODUIT ALIMENTAIRE FONCTIONNEL CONTENANT UN MÉLANGE DE FIBRES SPÉCIFIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.06.2007   FR 0756189**
**29.06.2007   US 947038 P**

(43) Date of publication of application:
**28.04.2010   Bulletin 2010/17**

(73) Proprietor: **Compagnie Gervais Danone**
**75009 Paris (FR)**

(72) Inventors:
• **AYMARD, Pierre**
**F-92160 Antony (FR)**
• **NOBLE, Olivier**
**F-91400 Orsay (FR)**

• **LYOTHIER, Arnaud**
**F-92600 Asnieres (FR)**

(74) Representative: **Warcoin, Jacques et al**
**Cabinet Regimbeau**
**20, rue de Chazelles**
**75847 Paris Cedex 17 (FR)**

(56) References cited:
**WO-A-2005/036971      WO-A-2006/134157**
**DE-A1- 19 943 188      FR-A- 2 858 630**
**US-A- 4 737 374      US-A- 4 971 810**

• **INTERNET CITATION, [Online] XP002215412 Retrieved from the Internet: URL:http: //www.nestleclinicalnutrition.com /product_ info_template.asp?ID=5> [retrieved on 2002-10-02]**

**Description**

The present invention relates to a fluid or semi-fluid food product, stable over time, containing a ternary mixture of viscosifying and non-viscosifying hydrosoluble fibres, and non-hydrosoluble fibres, and maintaining significant viscosity during digestion.

Gelling or thickening, hydrosoluble polysaccharide fibres have recognized health benefits.

In the stomach, hydrocolloids swell in the presence of water and induce gastric distension, increase bolus consistency and delay gastric emptying, which leads to a feeling of fullness and satiety.

In the present document, the term hydrocolloid refers to these texturing, hydrosoluble polysaccharide fibres, and therefore excludes starches which are also texturing polysaccharides but which are metabolised in the small intestine.

It is generally recognized that in the intestines, the increased viscosity that they generate:

- Reduces the rate of diffusion of nutrients, making it possible for example to reduce the rate of onset of post-prandial glucose, inducing limitation of the glycaemia peak which is useful for diabetics for example.
- Promotes passive excretion of biliary acids, which are carried in the viscous bolus. In return, the body re-synthesizes these biliary acids, which leads to cholesterol consumption and hence to a reduced level of circulating blood cholesterol.

The incorporation of gelling or thickening hydrocolloids in fluid to semi-fluid media nonetheless gives rise to major technological problems.

With respect to dairy products:

- It is impossible to incorporate significant quantities of hydrocolloids in the milk mix before fermentation: the limited compatibility between polysaccharides and milk proteins translates as phase separation, and the formation of poor quality curd, making the product unfit for consumption.
- The incorporation of hydrocolloids via a fruit preparation is also limited by the development of a texture in the fruit preparation that is too dense, making the fruit preparation too viscous and unpumpable.

For example, the maximum quantity of native guar which can be incorporated in stirred fruit yoghurt using conventional methods is in the order of 0.2%.

The use of "viscosity depressors", such as weakly hydrolysed maltodextrins, i.e. having a low dextrose equivalent (DE), or of non-viscosifying hydrosoluble fibres which can limit the viscosity of hydrocolloid solutions, may be a technical solution of interest. It is necessary however to use a large excess of these viscosity depressors compared with the quantity of viscosifying solubles fibre in order to obtain the desired effect. Under these conditions, some viscosity depressors raise problems, such as the formation of insoluble crystals which sediment during storage as with weakly hydrolysed maltodextrins, or with native or long-chain inulins. This crystallization causes a deterioration of the organoleptic quality of the product (gritty sensation in particular) and a colour change (e.g. strawberry fruit preparations turn from red to pink). These viscosity depressors may also have nutritional disadvantages such as a non-negligible calorie contribution (for maltodextrins) or symptoms of digestive discomfort due to fermentable fibres in the colon. Additionally, binary mixtures of the above-mentioned viscosifying and non-viscosifying soluble fibres are insufficiently stable over time. Phase separation of the product, of greater or lesser extent, is observed over time which may go as far as the onset of a highly textured phase side by side with a phase that has greater volume but is of much lower viscosity. This type of system therefore carries risks of instability, either on a day or week scale during storage at constant temperature, or on shorter time scales in the event of temperature change such as is the case with a fresh product stored at a temperature of between 4 and 10°C and whose temperature rises to 37°C after ingestion.

Patent application WO0067592 by Opta Food Ingredients shows that it is possible to use a maltodextrin with low DE, hydrolysed guar or inulin, to obtain a significant reduction in the viscosity of a glucomannan solution.

Similarly, patent application US2003013679 by Abbott Laboratories describes the use of low DE maltodextrin to reduce substantially the viscosity of a 2% guar solution.

However, to obtain the desired reduced viscosity effect, it is necessary to use a large excess of maltodextrins compared with the soluble viscosifying fibre. According to patent application US2003013679, the quantity of maltodextrins required lies between 5 to 14 times the quantity of soluble viscosifying fibres. For example, for a 2% concentration of soluble fibre in the end product, between 10% and 28% weakly hydrolyzed maltodextrins need to be added, which contributes a caloric content of 40 to 112 kcal per 100g of end product. In addition to their caloric value, identical to that of sugars (4 kcal/g), maltodextrins are produced by partial hydrolysis from fully gelatinised starch, and are therefore rapidly metabolised by digestive amylases (salivary and intestinal). This promotes the rapid onset of blood glucose, thereby countering the benefit of the soluble fibre content which is known to delay the onset of blood glucose. This technical solution is therefore of very limited interest when developing ingredients intended to manage weight or satiety.

As for inulin and the other more or less hydrolysed fructo-oligosaccharides, these show low gastro-intestinal tolerance, with the onset of symptoms of discomfort (flatulence, borborygmi, ...) over and above 15 to 20g/day. Having regard to the substantial quantities needed to obtain a reduced viscosity effect, the end product which can contain 10 to 15 g fructo-oligosaccharides, therefore has limited tolerance.

The article by Jasim Ahmed et al (Int. Journal of Food Properties, 2005, 8, 179-192) reports that the addition of gum acacia significantly reduces the rheology of guar (and xanthan gum). However, these observations relate to a very high proportion of gum acacia in the mixture, between 16 and 80 times that of guar gum. With low guar contents (0.25% mixed with 20% gum acacia) the viscosity is very close to that of gum acacia at this concentration. When the guar concentration increases up to 1.25%, the viscosity also increases strongly (exponential aspect) even if it remains lower than that of pure guar gum (i.e. in the absence of the 20% gum acacia). On reading this article, it is therefore impossible to extrapolate the behaviour of the guar/acacia mixture to proportions such as those concerned by the present invention. The modelling and conclusions given by the authors are effectively based only on tests conducted in an experimental domain removed from the area investigated below. The author of this publication did not wish to use a large quantity of guar gum, and had to focus on problems specific to confectionery which appear difficult to extrapolate to fluid or semi-fluid food products. In addition, this technical solution leads to a very high gum acacia content in the end product, hence risks of gastro-intestinal discomfort due to fermentation of the gum acacia in the colon: for a guar content of 2% in the end product, the acacia doses used according to Jasim et al. are higher than 32%. Very high doses of gum acacia in the end product are only tolerable if the quantities of consumed end product are low, in the order of a few grams, which is the case with confectionery (sweets, chewing-gum, etc...) . However, for fluid to semi-fluid products, such as fresh dairy products, beverages and fruit purees available in portions in the region of 100 g or more, the quantities of gum acacia mentioned in the article by Jasim et al carry intolerance risks for consumers.

Patent application WO 2005036971 mentions gum acacia gum *inter alia* as rheology modifier in systems comprising:

- at least 50% of an absorbable sugar
- a thickener such as guar, beta-glucan and/or modified starch.

The objective is to produce dry products (cereal products, pasta) with a reduced content of absorbable sugar. The problem solved by this document is therefore far removed from the problem of the present invention.

Patent EP 1008306 describes the addition of gum acacia which enables the viscosity of a psyllium solution to be reduced. The patent focuses on psyllium and does not mention any other texturizer, the best viscosity depressor claimed is a modified manioc starch. Again, it is necessary to add very high quantities of modified manioc starch to obtain a notable effect on viscosity. The viscosity of a 2% psyllium solution can be reduced practically to the value of a 1% psyllium solution by adding manioc starch, but with a quantity in the order of 10% to 20%.

Patent US 5 545 411 describes the addition of 1 to 2% gum acacia to reduce (by 20 to 30%) the viscosity of preparations for enteral nutrition containing soy fibres (0.2% to 3%).

Patents JP 2005185132 and US 4 988 530 describe beverages simultaneously containing pectin and gum acacia. However, no mention is made of a specific effect on viscosity contributed by gum acacia.

Patent US 4 971 810 describes a method to produce yoghurts containing fibres, in particular gum acacia. However, the problem of reducing viscosity is not approached.

The prior art therefore shows that the strong viscosity generated by viscosifying hydrosoluble fibres such as guar, can be reduced in the presence of certain molecules with high molecular weight, such as weakly hydrolysed maltodextrins, gum acacia, partly hydrolysed guars, modified starches, etc... However, these molecules need to be added in large excess i.e. a dose generally at least 10 times greater than that of the soluble viscosifying fibre.

The prior art further shows that these binary mixtures are used to formulate products having an effect on weight management, satiety or blood glucose control. As a general rule (cf. in particular US2003013679), the products described in the prior art are in the form of a powder mixture which the consumer re-hydrates before ingestion. However a powder mix, as conventionally proposed in the food supplement industry, is insufficient to produce a food product of fresh dairy product, beverage or fruit puree type. A food product with high water activity undergoes mechanical heat treatment at least to guarantee a certain microbiological quality, which may modify interactions between the two types of fibres. Additionally, the mixture of viscosifying fibres and viscosity depressor, once hydrated in the food product, must have chemical and physical stability throughout the lifetime of the product. In particular, any macroscopic phase separation derived from the succession of coalescence and creaming/sedimentation phenomena must be avoided. Curiously, the prior art does not mention the fact that binary mixtures of fibres may undergo rapid change, soon showing phase separation during storage.

The patent application WO 2006/134157 describes a fresh dairy product with satietogenic power based on viscosifying hydrosoluble fibres such as guar gum at least partly hydrolyzed.

However, partly hydrolyzed guar gum has an intrinsic viscosity at ambient temperature of 0.3 dl/g and therefore does not cause any viscosity and any stability problem and therefore is easier to carry out than native guar gum.

In particular, a composition of an intermediate preparation which is intended to be used in yogurt is given on table 4 of WO2006/134157.

This intermediate product contains 11 % by weight of SunFiber® which is a partly hydrolyzed guar gum.

It also contains 3 % by weight of wheat and 16 % by weight of apple puree. According to the book of Souci-Fachmann-Kraut (Food composition and nutrition Tables) and their online database available at www.sfk-online.net, apple puree contains 2 % by weight of total fibres. Within these fibres, 24 % by weight are hydrosoluble fibres wherein 76 % by weight are non-hydrosoluble fibres.

Therefore, the intermediate preparation described in this document contains only 0.0768 % by weight of non-viscosifying hydrosoluble polysaccharide fibres. 18 % by weight of this intermediate preparation is added in yogurt in order two obtain a yogurt which contains 2 g of guar gum per 125g of end product. Therefore, the yogurt contains only 0.0138 % in weight of non-viscosifying hydrosoluble polysaccharides fibres.

This document does not describe nor suggests that non-viscosifying hydrosoluble polysaccharide fibres could have an impact on the viscosity. In fact, the quantity used in this document of this type of fibres, is not enough to produce a real viscosity decrease. Furthemore, this decrease in viscosity is not needed since partly hydrolyzed guar gum does not cause any viscosity increase when used in a yogurt. Therefore, in this document, the non-viscosifying hydrosoluble polysaccharide fibres do not play the role of a "viscosity depressor".

Surprisingly, the inventors have discovered that it is possible to use non-viscosifying, hydrosoluble polysaccharide fibres as « viscosity depressor », such was gum acacia, in association with non-hydrosoluble cellulose fibres, so as to obtain a fluid, or semi-fluid food product that is stable over time end has a high content of viscosifying hydrosoluble polysaccharide fibres such as guar gum. In addition, the proportion of gum acacia needed to reduce the viscosity of guar gum (or the other soluble, viscosifying fibres) is very much lower than that mentioned in the prior art: a gum acacia content 1.5 to 4 times the content of guam gum is sufficient to obtain a signification seduction in viscosity. The presence of an insoluble, fibre is necessary however to guarantee the stability of the mixture and product homogeneity during storage.

Therefore, stable, semi-fluid aqueous "solutions" containing up to 5 weight % guar gum can be obtained using ternary mixtures (guar/acacia/non-hydrosoluble cellulose fibres) in adequate proportions. Similarly, it is possible to produce syrups for beverages which have guar gum concentration but remain sufficiently fluid so that they can be pasteurised and diluted using conventional equipment.

Also, some non-viscosifying, hydrosoluble polysaccharide fibres, such as gum acacia, fructo-oligosaccharides, hydrolysed guar, have a prebiotic effect. The fermentation of these fibres by colon flora produces short-chain fatty acids (butyrate, propionate, pyruvate) and locally decreases pH, these two actions leading to an increased Bifidobacterium population and reducing populations of pathogenic bacteria (Coliforms, Salmonellae, etc...). This fermentation can nevertheless lead to discomfort due to the production of gases in particular, and it is important to give consideration also to gastro-intestinal tolerance: for example, gum acacia is better tolerated than fructo-oligosaccharides (FOS): the dose for onset of minor symptoms of discomfort, such as flatulence, is 40g/day for gum acacia compared with 15-20g/day for FOS and inulins. The doses of gum acacia used in the present invention are lower than this acceptable dose, and therefore do not give rise to any problems of gastro-intestinal tolerance.

The present invention therefore relates to a stable, fluid or semi-fluid product whose dry extract is less than 30 weight % relative to the total weight of the product, advantageously less than 20 weight %, comprising between 1 and 24 weight % fibres relative to the total weight of the food product, characterized in that the fibres consist of a mixture of :

A) 0.4 to 5 % weight %, relative to the total weight of the product, of viscosifying, hydrosoluble polysaccharide fibres,
B) 0.8 to 20 weight %, relative to the total weight of the product, of non-viscosifying hydrosoluble fibres having a mean molar mass of between $3.10^5$ and $3.10^6$ g/mol and an intrinsic viscosity in an aqueous solution of less than 0.3 dl/g,
C) 0.04 to 0.6 weight %, relative to the total weight of the product, of non-hydrosoluble cellulose fibres.

Advantageously, said viscosifying hydrosoluble polysaccharide fibres have a mean molar mass equal to or greater than $7.10^5$ g/mol, and/or an intrinsic viscosity at ambient temperature of more than 5 dl/g, more advantageously an intrinsic viscosity at ambient temperature of more than 6 dl/g.

In the meaning of the present invention, by « viscosifying hydrosoluble polysaccharide fibres », are meant any native or weakly hydrolysed, hydrosoluble, polysaccharide food fibres which provide viscosity at a low dose.

Among these fibres, polysaccharides of mean molar mass equal to or greater than $7.10^5$ g/mol and of straight or slightly branched structure are called « viscosifying » insofar as their incorporation at low doses (typically between around 0.05 and 0.5%) can increase the viscosity of the solvent by several orders of magnitude. This effect is related to substantial osmotic swelling of the polymer chain in water, causing the assuming of an extended conformation, thereby mobilizing a high number of water molecules. The solution containing the viscosifying polymer has a slower flow rate and increased viscosity, viscosity being defined as the ratio between the exerted stress to generate flow and the characteristic speed of this flow. For objective quantification of the thickening nature of a polymer, it is advantageous to refer to the volume occupied by the polymer chain in solution: intrinsic viscosity by definition is the so-called occupied « hydrodynamic » volume per gram of polymer in solution. This volume can be determined experimentally, by measuring the viscosity of polymer solutions at different concentrations and extrapolating the value of reduced viscosity at zero concentration. Typically, native guars whose molecular weight is equal to or more than $10^6$ g/mol have an intrinsic viscosity in the order of 8 to 30 dl/g (Doublier, and Wood, Cereal Chemistry, 1995, 72, 335-340).

Advantageously, the viscosifying, hydrosoluble polysaccharide fibres of the present invention are of natural, plant origin and advantageously are chosen from among carouba gum, fenugreek, konjac glucomannan, tara gum, oat and barley beta-glucans, guar gum, pectin and orange pulp fibres. Further advantageously, they are chosen from among guar gum, carouba gum, konjac glucomannan and oat and barley beta-glucans. Particularly advantageously it is guar gum. Advantageously, the guar gum is the gum marketed under the trade name Meyproguar M 225 (Danisco) or Viscogum MP 41230 (Cargill). These are conventional, non-hydrolysed native guars.

Advantageously, in the food product of the invention, the ratio between the proportions of guar gum and gum acacia is around 1.5 to 6. Preferably, this ratio is around 2 to 4.

In the meaning of the present invention, by « non-viscosifying hydrosoluble polysaccharide fibres », are meant any polysaccharide hydrosoluble food fibres which do not give viscosity at low dose despite their high molar mass (between $3.10^5$ and $3.10^6$ g/mol). These fibres have a very compact conformation and occupy a small hydrodynamic volume in solution, which translates as low viscosity in solution.

Advantageously, they are chosen from among gum acacia, hydrosoluble apple fibres (Pomelite LV® for eample) or soy fibres (Soya Fibe® for example), advantageously from among gum acacia and hydrosoluble apple fibres, further advantageously it is gum acacia. Gum acacia is one of the soluble fibres having most interest from a technological (weakly texturing gum) and nutritional viewpoint (good digestive tolerance and prebiotic effect). Gum acacia is a natural, soluble food fibre. It is a macromolecule with high molar mass (between $4.10^5$ and $2.10^6$ g/mol) but whose intrinsic viscosity is less than 0.2 dl/g (Al-Assaf et al, Food Hydrocolloids, 2005, 19, 647-667 ; Flindt et al, Food Hydrocolloids, 2005, 19, 687-701). For an equivalent molar mass, the intrinsic viscosity of gum acacia is 30 to 40 times lower than that of guar, which demonstrates the different conformation in solution. Gum acacia, also known as gum arabic, is an acacia exudate, solely purified using a physical process well known to those skilled in the art, consisting of the steps of grinding, dissolving in water, filtering, centrifuging, microfiltration, then spray drying or granulation. There are two types of gum acacia: *acacia seyal* and *acacia senegal.* Their structure is slightly different. They can however be distinguished by a very different rotating power and by their proportion of simple sugars (46% arabinose in *acacia seyal* and 24 % in *acacia Senegal).* Advantageously, the gum acacia is *acacia senegal, acacia seyal* or their mixture. By *« acacia senegal »* gum is meant a gum produced from natural exudates or produced by tapping stems or branches of trees of genus *Acacia senegal.* Advantageously, the gum acacia is Fibregum B from CNI.

Advantageously, the non-hydrosoluble cellulose fibres contain cellulose and/or hemi-cellulose. Advantageously, they are chosen from among wheat, cotton, wood fibres and their mixture, advantageously they are wheat fibres.

Advantageously, the non-hydrosoluble cellulose fibres and the non-viscosifying hydrosoluble polysaccharide fibres are in the form of a close mixture, advantageously obtained ($\alpha$1) by co-drying non-hydrosoluble, cellulose fibres with non-viscosifying hydrosoluble polysaccharide fibres, or ($\alpha$2) by mixing under strong shear, advantageously of more than $10^4$ s$^{-1}$, non-hydrosoluble cellulose fibres with non-viscosifying hydrosoluble polysaccharide fibres, or ($\alpha$3) by homogenizing under pressure, advantageously of at least 50 bars, a mixture of non-hydrosoluble cellulose fibres and non-viscosifying hydrosoluble polysaccharide fibres. Advantageously, it is a co-dried mixture obtained at step ($\alpha$1). Advantageously, this mixture contains between 5 and 30 weight % non-hydrosoluble cellulose fibres relative to the total weight of the mixture, advantageously 20 weight %, and between 70 and 95 weight % non-viscosifying hydrosoluble polysaccharide fibres relative to the total weight of the mixture, advantageously 80 weight %. Over and above 30% of non-hydrosoluble cellulose fibres, the mixture could not be dried. Below 5% non-hydrosoluble cellulose fibres, this mixture would have no use.

It is the non-hydrosoluble cellulose fibre which acts as stabilizer in the food product according to the invention. However, it only develops its function after being dispersed, and this dispersion takes place by means of the non-viscosifying hydrosoluble polysaccharide fibres in the homogenous mixture. Therefore, it can be considered that it is the homogeneous mixture which acts as stabilizer in the food product of the invention. Advantageously, the non-hydrosoluble cellulose fibres are wheat fibres Advantageously, the co-dried mixture used is a wheat fibre/gum acacia system marketed by CNI under the trade name "Equacia®".

In the meaning of the present invention, by « shear » is meant a shear rate advantageously expressed in s$^{-1}$.

In the meaning of the present invention, by « fluid or semi-fluid food product », is meant a product which can be drunk directly from a bottle (fluid product) or sucked by applying moderate pressure on a sachet (fruit puree type) or carton, or which can be consumed using a spoon (semi-fluid product). Advantageously, the apparent viscosity of this product at a shear rate of a 10 s$^{-1}$ and at 20°C is between 0.05 Pa.s (for the most fluid products) and 10 Pa.s for the more textured products.

Advantageously, it is chosen from among fresh dairy products, plant juices, beverages and their mixtures, advantageously from among fruit fresh dairy products, fruit and/or vegetable juices, flavoured water or fruit purees. By « beverage » under the present invention is meant any product consisting chiefly of water and with flavouring, in particular flavoured water.

Advantageously, the fluid or semi-fluid food product of the intention is a fresh dairy product. Advantageously it is a fermented dairy product. This food product may for example be a dairy product in which a fruit juice or a soy juice is added.

By "fermented dairy product", is particularly meant a fermented dairy product ready for human consumption i.e. a fermented milk food. In the present application, fermented milks and yoghurts are more particularly concerned. Said fermented dairy foods can alternatively be "fromages blancs" or "petits-suisses".

The terms "fermented milk » and « yoghurt » are given their usual meaning in the dairy industry i.e. products intended for human consumption derived from acidifying lactic fermentation of a milk substrate. These products may contain secondary ingredients such as fruit, plants, sugar, etc. Reference can be made for example to French decree n°88-1203 of 30 December 1988 on fermented milks and yoghurts, published in Journal Officiel de la République Française dated 31 December 1988. Reference can also be made to "Codex Alimentarius " (prepared by the Codex Alimentarius Commission under the aegis of the FAO and WHO and published by the FAO Information Division, available on line at http://www.codexalimentarius.net; cf. more particularly volume 12 of the Codex Alimentarius "Codex standards for milk and dairy products ", and the standard " CODEX STAN A -1 1(a)-1975 ").

The term « fermented milk » is therefore reserved in the present application for a diary product prepared with a milk substrate which has undergone treatment at least equivalent to pasteurisation, seeded with microorganisms belonging to the species characteristic of each product. A « fermented milk » has not undergone any treatment to remove a constituent element of the milk substrate used, and in particular has not undergone coagulum decanting. The coagulation of « fermented milks » must not be obtained by means other than those which result from the activity of the microorganisms used. In practice, the term « fermented milk » is therefore generally used to designate fermented milks other than yoghurts and, depending on the country can be called "Kefir", "Kumiss", "Lassi", "Dahi", "Leben", "Filmjôlk", "Villi", "Acidophilus milk" for example.

The term « yoghurt » is reserved for fermented milk obtained, according to constant local usage, by the development of specific thermophilic lactic bacteria called *Lactobacillus bulgaricus* and *Streptococcus thermophilus,* which must be in living form in the end product, to the proportion of at least 10 million bacteria per gram relative to the milk part. In some countries, regulations authorize the addition of other lactic bacteria to yoghurt production, notably the additional use of strains of *Bifidobacterium* and/or *Lactobacillus acidophilus* and/or *Lactobacillus casei.* These additional lactic strains are intended to impart various properties to the end product, such as the property of promoting equilibrium of intestinal flora or modulating the immunity system.

The quantity of free lactic acid contained in the fermented milk substrate must not be less than 0.6 g per 100 g when sold to the consumer, and the protein content of the milk part must not be less than that of normal milk.

The term « fromage blanc » or « petit suisse » in the present application is reserved for a non-salted, non-matured cheese which has undergone fermentation solely with lactic bacteria (no fermentation other than lactic fermentation). The dry matter content of fromages blancs can be lowered down to 15 g or 10 g per 100 g of fromage blanc, depending on whether the fat content is 25% more than 20g, or no more than 20g per 100g of fromage blanc, after complete desiccation. The dry matter content of a fromage blanc lies between 13 and 20 %. The dry matter content of a petit-suisse is not less than 23 g per 100 g of petit-suisse. It is generally between 25 and 30 %. « Fromages blancs » and « Petits suisses » are generally grouped together under the name « Fromages frais » conventionally used in the technical area of the present invention.

Advantageously, the fresh dairy product is chosen from among yoghurts, including stirred yoghurts, yoghurt drinks, fromages frais and fermented milks.

In a particular embodiment, the fresh dairy product of the invention has low fat and sugar content.

In the meaning of the invention, a product is « low in fat » if it contains:

- less than around 3 g fat per 100 g of product if the product is a solid (of firm yoghurt or fromage frais type);
- less than around 1.5 g fat per 100 ml of product for a liquid product (of yoghurt drink type).

In this respect, the Applicant specifies that above definition complies with the Codex Guidelines for the Use of Nutrition Claims adopted by the Codex Alimentarius Commission in 1997 and amended in 2001.

A product « low in sugar » or « with low sugar content » is such that it does not contain more than:

- around 0.5 g sugar per 100 g of product for a solid product ;
- around 2.5 g sugar per 100 ml for a liquid product.

Here again, the Applicant points out that this definition complies with the Opinion given by the Inter-ministerial Commission on particular food products, dated 8 July 1998 and concerning the non-misleading nature of nutritional claims.

Advantageously, it is a product having low energy density. By « low energy density » product is meant here a product providing around 40 to 120 kcal per 100 g, preferably around 60 to 110 kcal per 100 g, further preferably around 70 to 100 kcal per 100 g.

In another embodiment, the fresh dairy product of the invention contains between 2 and 10 weight % proteins relative to the total weight of the fresh dairy product, advantageously between 4 and 7 weight % proteins. Advantageously, these proteins are milk and/or plant proteins. Milk proteins for example are chosen from among milk powder, caseins and serum proteins. Plant proteins for example comprise soy proteins and/or wheat proteins, in particular gluten and partly hydrolysed gluten.

Advantageously, the fresh dairy product of the present invention contains fruits. Advantageously the fruit is chosen from the group consisting of apple, orange, red fruits, strawberry, peach, apricot, plum, raspberry, blackberry, red currant, lemon, grapefruit, banana, pineapple, kiwi, pear, cherry, coconut, passion fruit, mango, fig, rhubarb, melon, exotic fruit, litchi, grapes, blueberry or their mixtures.

In a particular embodiment, the food product of the invention is a fresh dairy product with fruit in which the weight content, relative to the total weight of the product, of each of the fibres is the following:

A1) between 0.4 and 2%, advantageously 1%, of viscosifying hydrosoluble polysaccharide fibres,

B1) between 0.8 and 8%, advantageously 2%, of non-viscosifying, hydrosoluble polysaccharide fibres, and

C1) between 0.04 and 0.25%, advantageously 0.1% of non-hydrosoluble cellulose fibres.

In another embodiment, the fluid or semi-fluid food product of the invention is a fruit puree. Advantageously it contains:

A2) between 1 and 5 weight %, advantageously 2.5 weight %, relative to the total weight of the puree or fruit preparation, of viscosifying hydrosoluble polysaccharide fibres;

B2) between 2 and 20 weight %, advantageously 7.4 weight %, relative to the total weight of the puree or fruit preparation, of non-viscosifying hydrosoluble polysaccharide fibres, and

C2) between 0.05 and 0.6 weight %, advantageously 0.15 weight %, relative to the total weight of the puree or fruit preparation, of non-hydrosoluble cellulose fibres.

These fruit purees have a texture close to that of standard purees.

Advantageously, the food product of the invention is stable for at least 4 weeks at 4°C, advantageously for 12 months at room temperature. In the meaning of the present invention, by « stable food product » is meant a food product such as defined above having less than 5 weight % separated liquid phase after 8 weeks at 10°C, relative to the total weight of the food product, advantageously less than 3 weight % relative to the total weight of the food product, advantageously less than 1 weight % relative to the total weight of the food product. By « separated liquid phase » is meant the transparent aqueous phase appearing at the bottom of the food product. A product having less than 5 weight % separated liquid phase is considered to be a stable product since there is practically no macroscopic phase separation. A period of 4 weeks is the minimum period of stability expected for yoghurt.

Advantageously, the food product of the invention has a viscosity of more than 0.2 Pa.s, i.e. 200 times the viscosity of water, preferably under digestion conditions. Advantageously, the food product of the invention has a viscosity of more than 1 Pa.s, i.e. 1000 times the viscosity of water. Further preferably, the food product of the invention has a viscosity of more than 3 Pa.s, i.e. 3000 times the viscosity of water.

By « digestion conditions » is meant the time at which the food product is subjected to gastric and intestinal conditions i.e. at the pH of the stomach and intestines and in contact with digestive enzymes naturally present in these parts of the digestive tube. More precisely, these gastric and intestinal conditions can be mimicked *in-vitro* by placing said food product according to the invention in a beaker, then heating it to 37°C for 30 minutes followed by acidification to pH2 using a 4N HCl solution. After continual stirring for 10 minutes, 1.25 weight % pepsin is added in powder form to the product acidified during the preceding step. After waiting 35 minutes, the pH of the product is brought up to 6 using concentrated 4N sodium hydroxide (NaOH). After stirring for 10 minutes, 0.5 weight % powder pancreatin is added. Stirring is continued for 10 minutes.

The viscosity values given above are values measured under these conditions at a shear rate of 10 s[-1].

More details on this *in vitro* test are given below under example 3.

The present invention also relates to a method to produce a fluid or semi-fluid food product according to the invention comprising the following steps :

a) adding fibres according to the invention to an initial matrix, and

b) mixing the product obtained.

By « initial matrix » is meant milk, fermented milk, plant juice, fermented plant juice, water, fermented water, fruit puree.

Advantageously, the method of the invention comprises a step (α), prior to step (a), to disperse the non-hydrosoluble cellulose fibres in the non-viscosifying hydrosoluble polysaccharide fibres, by:

- α1)co-drying the non-hydrosoluble cellulose fibres with the non-viscosifying hydrosoluble polysaccharide fibres, or
- α2)mixing under strong shear, advantageously more than $10^4$ s$^{-1}$. the non-hydrosoluble cellulose fibres with the non-viscosifying hydrosoluble polysaccharide fibres, or
- α3)homogenizing under pressure, advantageously of at least 50 bars, a mixture of non-hydrosoluble cellulose fibres with non-viscosifying hydrosoluble polysaccharide fibres.

Advantageously, step (α) consists of step (α1), i.e. co-drying the non-hydrosoluble cellulose fibres with the non-viscosifying hydrosoluble polysaccharide fibres.

If co-drying is performed, in the end dried product there is preferably a maximum proportion of 30 weight % non-hydrosoluble cellulose fibres. Advantageously, the end dried product contains between 5 and 30 weight % non-hydrosoluble cellulose fibres, advantageously wheat fibres, relative to the total weight of the mixture, and between 70 and 95 weight % of non-viscosifying hydrosoluble polysaccharide fibres, advantageously gum acacia, relative to the total weight of the mixture, advantageously 80 weight % non-hydrosoluble cellulose fibres and 20 weight % non-viscosifying hydrosoluble polysaccharide fibres.

In an advantageous embodiment, the fibres added at step (a) are in the form of an intermediate preparation, advantageously chosen from among fruit preparations and syrups.

In the meaning of the present invention, by « fruit preparation » is meant any aqueous suspension containing fruit pieces or fruit puree. In the meaning of the present invention, by « fruit puree » is meant a fermentescible but non-fermented product obtained by sieving, or by another process, the comestible part of whole or peeled fruit without removing the juice. The puree may be concentrated and in this case is obtained from the fruit puree by physical removal of a determined part of the constituent water.

Advantageously, the fruit preparation of the invention has a texture, measured using a CENCO texture measurement system, of between 5 and 15, preferably between 5 and 12. This type of texture measurement is routinely used by those skilled in the art. It is not possible in this case to use conventional equipment to measure viscosity or texture, since the fruit preparations are not homogeneous mixtures.

The higher the CENCO measurement, the more the preparation is liquid.

In an advantageous embodiment, the fruit preparation of the invention also comprises sugar or a sweetener and optionally a colouring, flavouring agent and/or acidifier. The sugars are particularly monosaccharides and disaccharides. Amongst the monosaccharides, mention may be made of fructose, galactose, glucose. Amongst the disaccharides, particular mention may be made of sucrose.

In the meaning of the present invention, by « syrup » is meant a liquid preparation containing sugar, texturizer(s), water and flavouring(s).

The present invention also relates to an intermediate preparation, advantageously intended to be used in a fluid or semi-fluid food product of the invention, containing:

A3) between 2 and 10 weight %, advantageously 5 weight %, relative to the total weight of the intermediate preparation, of viscosifying hydrosoluble polysaccharide fibres,

B3) between 4 and 40 weight % , advantageously 10 weight %, relatives to the total weight of the intermediate preparation, of non-viscosifying hydrosoluble polysaccharide fibres, and

C3) between 0.2 and 1.25 weight %, advantageously 0.5 weight %, relative to the total weight of the intermediate preparation, of non-hydrosoluble cellulose fibres.

Advantageously, the intermediate preparation of the invention is a fruit preparation or a syrup.

The present invention additionally concerns the use of the intermediate preparation of the invention in a fluid or semi-fluid food product, advantageously in a fresh dairy product. It can in particular, in the case of a fruit preparation, be incorporated in a fermented dairy product, as a mixture or as a twin layer.

The present invention also relates to a method to produce an intermediate preparation according to the invention comprising the following successive steps:

- mixing the different fibres in powder form,
- dispersing this mixture of powders in water under stirring,

- optionally, adding fruit, sugar, colouring agents, flavours to this dispersion,
- subjecting the preparation obtained to pasteurising heat treatment,
- cooling the intermediate preparation obtained,
- storing the intermediate preparation in a low temperature container (lower than 10°C, preferably lower than 4°C).

The fruits are added in the form of puree, fruit pieces, juice, etc...

Advantageously, the non-hydrosoluble cellulose fibres are treated so as to promote their dispersion, by:

- α1)co-drying the non-hydrosoluble cellulose fibres with the non-viscosifying hydrosoluble polysaccharide fibres, or
- α2) mixing, under strong shear, advantageously at more than $10^4$ s$^{-1}$, the non-hydrosoluble cellulose fibres with the non-viscosifying hydrosoluble polysaccharide fibres, or
- α3) homogenizing under pressure, advantageously of at least 50 bars, a mixture of non-hydrosoluble cellulose fibres with non-viscosifying hydrosoluble polysaccharide fibres.

The ingredients of the intermediate preparation according to the invention, with the exception of the non-viscosifying hydrosoluble fibres and the non-hydrosoluble cellulose fibres of the invention, are for example viscosifying hydrosoluble polysaccharide fibres, fruit - in the form of puree of fruit pieces, acids, sugars or sweeteners, colouring agents, etc...

The conditions for pasteurising heat treatment are known to those skilled in the art.

Finally, the present invention relates to the non-therapeutic use of the food product according to the invention as satiating food to increase the sensation of gastric fullness, to delay the onset of feelings of hunger and/or to manage a person's body weight.

The term « satiating » such as used here meets the definitions usually recognized in this area. This notion has been the subject of an increasing number of publications. For information it is specified that by « satiating food » is meant here a food which, for the consumer, particularly leads to a reduced feeling of hunger, reduced appetite, increased gastric fullness, delayed return of hunger between two food intakes, increased time interval between two food intakes, reduced food intakes after ingestion. These different effects can be observed alone or in association, in full or in part. It is also recalled that there are marker measurement methods which can be used to determine the satiating property of a food as described below (see in particular Table 1). In particular, satiating foods contribute towards the release of pre- and post absorbing signals which take part in controlling gastric kinetics, pancreatic secretion and food intake. The actions of these signals act at peripheral and central level (see Table 1). Table 1 below summarizes the most frequent markers. For more information on these markers, see the review made by De Graaf et al, 2004.

**Table 1**

| MARKERS | HUNGER SATISFACTION (end of meal) | SATIETY (start of meal) |
|---|---|---|
| Behavioural | Food intake | Previous food intake |
| | | Time interval between meals |
| | Subjective determination of appetite (e.g. hunger and feeling of gastric fullness) | Subjective determination of appetite (e.g. hunger and feeling of gastric fullness) |
| Peripheral | Stomach distension | Change in plasma level of blood glucose (ST) |
| | Measurement of plasma CCK (Cholescystokinine) | Measurement of plasma leptin (LT) |
| | Measurement of plasma GLP-1 (Glucagon-like peptide-1) | Measurement of plasma ghrelin (ST & LT) |
| Central | Brain image | Brain image |
| ST: short-term LT: long-term | | |

With the invention, satiating products can be formulated owing to the high content of viscosifying soluble fibres: between 0.4% and 4% relative to the weight of the end product. At these doses, the viscosity of the guar gum lies between 0.2 and 150 Pa.s (i.e. between 200 times and 150 000 times the viscosity of water at 25°C). Viscosity is measured using a high precision MCR300 rheometer by Anton Paar - Physica, using a CC27 coaxial cylinder and a measurement chamber thermostat-regulated by Peltier effect (TEZ 150 PC). The chosen shear rate was 10 s$^{-1}$, known

to represent shears encountered under intestinal conditions.

Under the effect of these viscosifying fibres, the food bolus becomes highly thickened, which delays gastric emptying and bolus advance in the intestine, thereby contributing towards the satiating effect.

The fact that is of interest in our case is that the system consisting of viscosifying fibres having satiating effects and of non-viscosifying fibres having prebiotic effects does not develop its viscosity in the product, or only to a very small extent, but it ensures its function(s) as soon as it arrives in the digestive tract.

The satiating viscosifying fibre (e.g. guar gum) acts rather more in the high digestive sphere (stomach and small intestine) whilst the prebiotic non-viscosifying fibre (e.g. gum acacia) acts rather more in the low digestive sphere (large intestine and colon).

The invention also relates to the non-therapeutic use of the food product according to the invention as a food, preferably a functional food, reducing a person's circulating blood cholesterol and delaying glycaemic and insulinaemic response of the person's body after a meal.

It also relates to the non-therapeutic use as functional food which reduces circulating blood cholesterol.

The present invention also relates to the use of the food product according to the invention as medicinal product.

Preferably the food product according to the invention is used as medicinal product and as functional food which reduces a person's circulating blood cholesterol and delays such person's body glycaemic and insulinaemic reponse after a meal, thereby preventing the onset of symptoms of metabolic syndrome.

According to another aspect of the invention, the food product of the invention is used as medicinal product and as functional food reducing a person's circulating blood cholesterol and preventing the onset of symptoms related to cardiovascular disorders.

The invention will be better understood in the light of the following figures and non-limiting examples.

Figure 1 shows measurement of viscosity in Pa.s of an aqueous solution of native guar gum (Meyproguar M225) at a shear rate of $10s^{-1}$ in relation to the concentration of native guar gum (weight %).

Figure 2 shows measurement of viscosity in Pa.s of an aqueous solution of native guar gum at a shear rate of $10s^{-1}$ in relation to the concentration of native guar gum (weight %) and in the presence of a certain quantity of gum acacia (0.10 or 20 weight °).

Figure 3 shows measurement of viscosity in Pa.s of an aqueous solution of native guar gum at a shear rate of $10s^{-1}$ in relation to the concentration of native guar gum (weight %) in the presence of a constant proportion of gum acacia relative to the quantity of guar: the guar/acacia ratio varies between 1.5 and 4. In figure 3, the quantities of guar and acacia are therefore both variable, but their ratio remains constant.

Figure 4 shows the concentration of gum acacia (weight %) in an aqueous solution, in relation to the concentration of guar gum (weight %) so that viscosity equals 10 Pa.s at a shear rate of $10s^{-1}$.

Figure 5 shows measurement of viscosity at a shear rate of $10s^{-1}$ (in Pa.s) of an aqueous solution containing 2 weight % native guar gum and 10 or 20 weight % of different non-viscosifying hydrosoluble fibres: gum acacia (Fibregum B), apple (Pomelite) and soy (Soyafibe).

Figure 6 illustrates the measurement of viscosity at a shear rate of $10s^{-1}$ (in Pa.s) of an aqueous solution containing 2 weight % native guar gum (Meyproguar M225, Danisco) or 2 weight % glucomannan (Rheolex RS, Shimizu), or 5 weight % pectin (TS-P 6786, Danisco) or 5 weight % beta-glucan enriched oat bran (Oatwell 22, CreaNutrition). The viscosity of these viscosifying soluble fibres is compared without gum acacia and with the addition of 10 % gum acacia (Fibregum B).

Figure 7 shows an example of *in vitro* test results for apparent viscosity (in Pa.s) at 37°C and at a shear rate of $10s^{-1}$ in relation to the digestion step obtained with a low-fat, stirred yoghurt of Taillefine Brassé Nature type, and with the same yoghurt mixed with a fruit preparation containing 5 weight % native guar gum and 10 weight % gum acacia. The proportion of white mass and fruit preparation being 80 and 20 weight % respectively in the end product, the quantities of native guar gum and gum acacia are respectively 1 weight % and 2 weight % in the end product.

Viscosity is measured using a high precision MCR300 rheometer by Anton Paar - Physica, using a CC27 coaxial cylinder and a measurement chamber thermostat regulated by Peltier effect (TEZ 150 PC). The chosen shear rate is 10 $s^{-1}$, which is known to represent shear conditions encountered under intestinal conditions.

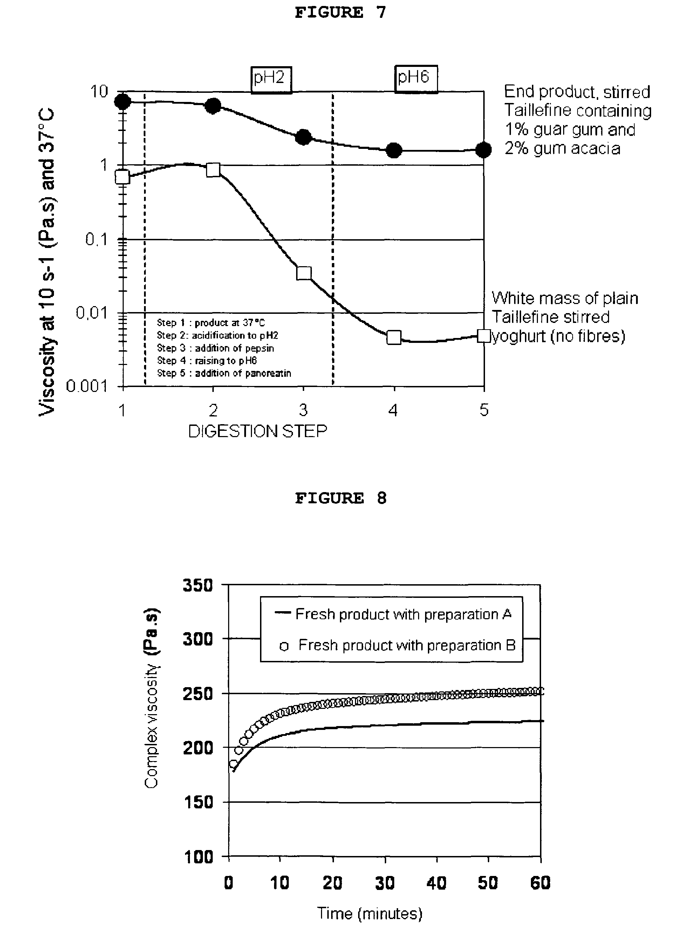

Figure 8 shows changes over time in the complex viscosity of mixtures consisting of 80% hyper-protein white mass (containing 6.5 % protein) and 20 % fruit preparation A (containing 7.5% guar gum and 20% gum acacia), or a fruit preparation B (containing 7.5% guar gum and 20% gum acacia). Product A therefore contains 1.5% guar gum and 2.5% gum acacia, and product B contains 1.5% guar gum and 4% gum acacia.

**Example 1: Preparation of semi-fluid « solutions » containing 2% to 4% guar gum, in the presence of gum acacia and wheat fibres.**

The guar used is the reference Meyproguar M 225 (Danisco). It is a conventional native guar, non-hydrolysed. Its molar mass is 2.7 $10^6$ g/mol, which corresponds to an intrinsic viscosity in the order of 20 dl/g (Doublier and Wood, Cereal Chemistry, 1995, 72, 335-340). The gum acacia used is Fibregum B (CNI). Its molar mass is 6.4 4 $10^5$ g/mol, which corresponds to an intrinsic viscosity in the order of 0.18 dl/g (Al-Assaf et al, Food Hydrocolloids, 2005, 19, 647-667 ; Flindt et al, Food Hydrocolloids, 2005, 19, 687-701).

Laboratory tests were conducted under the following conditions:

-    mixing the powders and dispersing in cold water,
-    heating the mix, under stirring (shear 30 $s^{-1}$) up to 95°C,
-    cooling under stirring (shear 30 $s^{-1}$) down to 20°C,
-    measuring apparent viscosity at a shear rate of 10 $s^{-1}$, using a MCR300 rheometer (Anton Paar) equipped with coaxial cylinders (CC27).

Under these conditions:

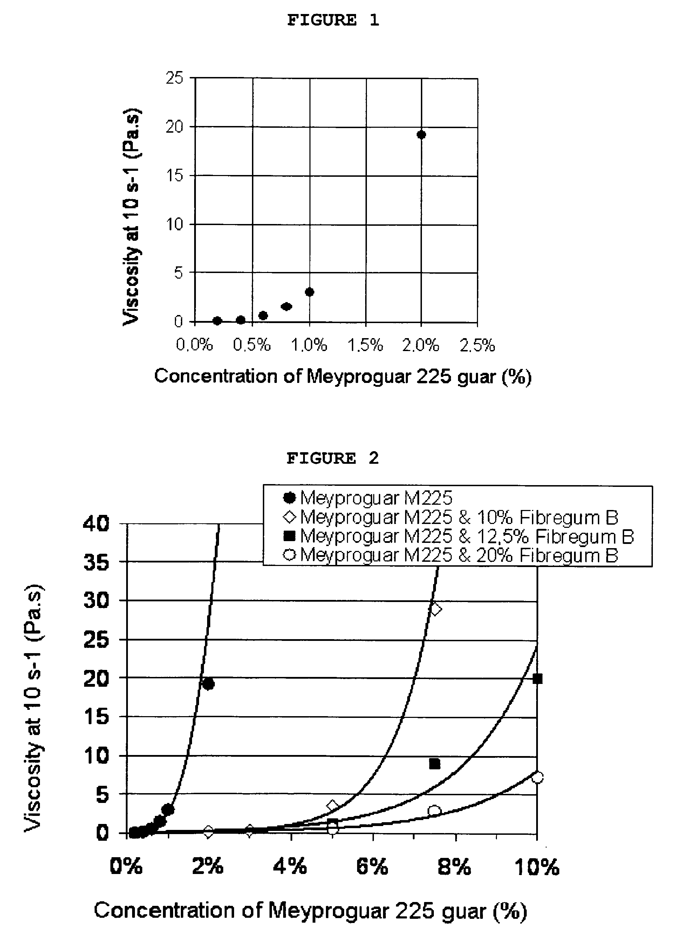

The solutions of guar alone rapidly develop high viscosity (see Figure 1). At a concentration of 2 weight % guar, the viscosity already reaches 19 Pa.s, i.e. a solution difficult to pump. To obtain a semi-fluid « solution » in the meaning of the invention, the maximum dose of guar which can be incorporated is around 1.5 weight %.

Solutions of gum acacia alone have low viscosity: at 20% the viscosity of a solution of gum acacia is only 0.02 Pa.s, i.e. 20 times the viscosity of water at 25°C (Jasim Ahmed et al, Int. Journal of Food Properties, 2005, 8, 179-192).

In the presence of a sufficient concentration of gum acacia, it is possible to obtain semi-fluid « solutions » containing up to 10 weight % guar gum (Figure 2).

They are in fact two-phase systems in which the following exist side by side:

-    a continuous phase essentially containing gum acacia (and a small quantity of guar),
-    a dispersed phase rich in guar and containing a small quantity of gum acacia.

These two phases have a different density, and for reasons of stability, it is necessary to add non-hydrosoluble cellulose fibres to the product, such as wheat fibres.

It therefore appears possible to obtain semi-fluid « solutions » containing 0.4 to 5 weight % guar gum, in the presence of gum acacia at a concentration of 0.8 to 20 weight %.

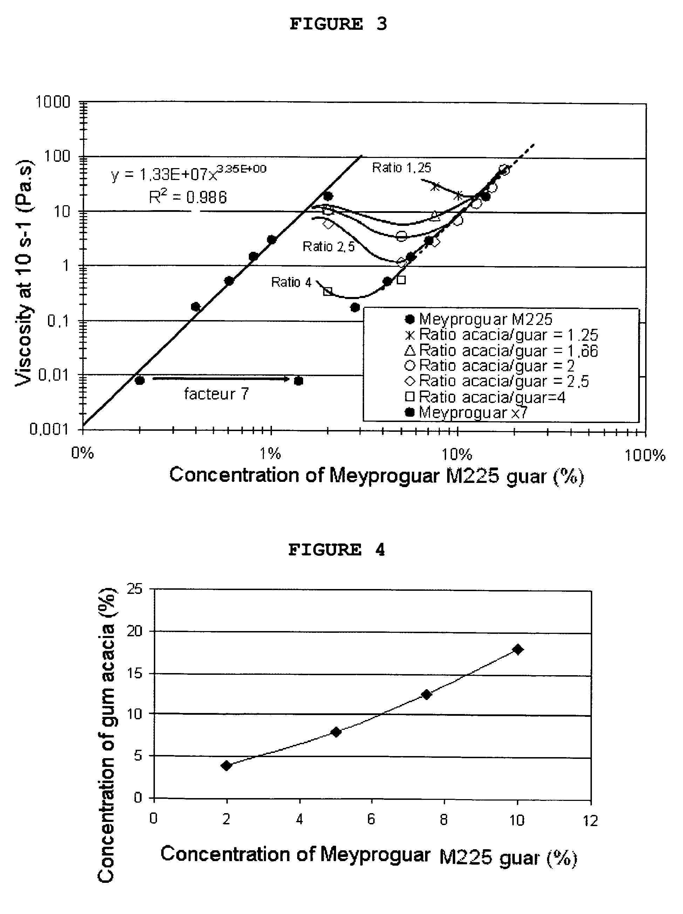

Figure 3 also shows the viscosity in Pa.s of an aqueous solution of native guar gum at a shear rate of 10$s^{-1}$, in relation to the concentration of native guar gum (weight %) and gum acacia added in a constant proportion relative to the quantity of guar. The solid symbols correspond to changes in apparent viscosity at 10$s^{-1}$ in relation to the concentration of guar alone: viscosity increases markedly with concentration, which can be adjusted by a power rule, to the power of 3.35. In the presence of acacia, added proportionally to the quantity of guar gum, this same power rule behaviour is observed but with much greater guar contents and which varies according to the acacia/guar ratio. For example, for an acacia/guar ratio of 2.5, the power rule behaviour is found for guar gum concentrations of more than 6% (and hence acacia concentrations of more than 15%). For an acacia/guar ratio of 4, this behaviour is found for concentrations in the order of 3 to 4%.

Below these critical concentrations, the viscosity of the guar/acacia mixture changes complex fashion and increases even with dilution: for example it increases from a value of 1.2 Pa.s for a guar concentration of 5% and acacia concentration of 12.5%, to a value of 6.0 Pa.s for a guar concentration of 2% and acacia concentration of 5%: this increase in viscosity with dilution is fully particular to the invention and is never observed with a single viscosifying fibre.

In figure 3, the shaded circles represent the values of guar gum alone, but at concentrations multiplied by 7. The points obtained coincide well with the trend in viscosity in relation to the concentration of the acacia/guar mixtures, at least for the highest values. The mixture of fibres according to the invention therefore allows the incorporation in practice of up to seven times more viscosifying fibres in the end product, whilst maintaining the same viscosity.

This confirms the interest of the invention for formulating health products:

a) it is possible to obtain an end product rich in fibres but having limited viscosity in its packaged form, compared with the viscosity which would be generated by the viscosifying soluble fibres alone.
b) after ingestion, the product is gradually diluted during digestion, either by drinking water or by digestive fluids (salivary, gastric juices, etc...). As shown in figure 3, the viscosity remains high during dilution, and can even increase over a certain concentration range, in relation to the acacia/guar ratio.

For a given concentration of guar gum, there is a minimum concentration of gum acacia with which it is possible to obtain a system having a viscosity of less than 10 Pa.s, which corresponds to a product that is easy to pump and mix. The relation between these two concentrations is given figure 4. The area above the line corresponds to the area of possible functioning. Globally, the quantity of gum acacia to be used is 2 to 3 times the quantity of targeted guar. Again, this factor of 2 to 3 is significantly lower than those mentioned in the prior art for viscosity depressors, in which the ratio is close to 10.

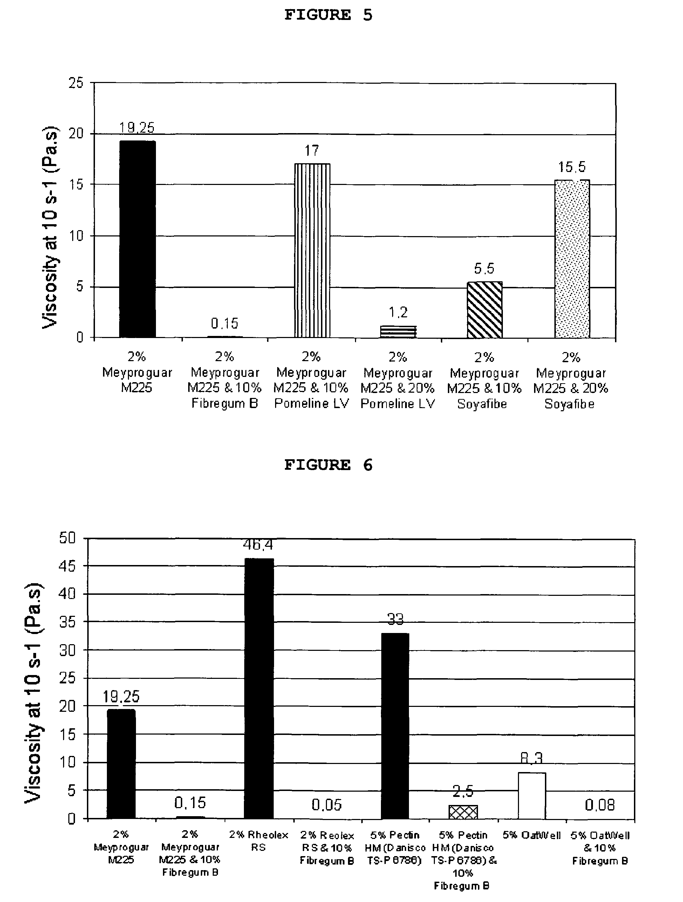

Similar results, but of lesser interest, can be obtained using soy fibres (Soya Fibe, supplier: Fuji Oil) or soluble apple fibres (Pomelite LV, supplier: Val de Vire). (Figure 5):

- It is possible to reduce 20 times the viscosity of a 2 weight % solution of native guar gum with 20 weight % soluble apple fibres (Pomelite LV).
- It is possible to reduce 4 times the viscosity of a 2 weight % solution of native guar gum with 10 weight % soy fibres (Soya Fibe).

Similar results, and of similar interest, are also obtained using gum acacia to reduce considerably the viscosity of other viscosifying fibres in solution (figure 6):

- It is possible to reduce 100 times the viscosity of a 2 weight % solution of Glucomannan (Rheolex RS supplier: Shimizu) with 10 weight % gum acacia.
- It is possible to reduce 10 times the viscosity of a 5 weight % solution of Pectine HM (Grinsted pectin TS -P 6786 supplier: Danisco) with 10 weight % gum acacia.
- It is possible to reduce 100 times the viscosity of a 5 weight % solution of $\beta$-glucan enriched oat bran (Oatwell 22 wt. % $\beta$-glucane supplier: Crea Nutrition) with 10 weight % gum acacia.

**Example 2: fruit preparations rich in guar gum**

The method described in example 1 can be used to obtain fruit preparations containing 1 to 10 weight % guar gum.

By way of example, a strawberry preparation free of sugar containing 5 weight % guar gum was prepared under the following conditions:

Composition :

- Concentrated strawberry puree (x6)        8.3%
- Guar gum (Meyproguar M 225)        5.0%
- Gum acacia (Fibregum B)        6.0%
- Equacia®        5.0%
- Water        74.3%

+ Acidifiers, sweeteners, colourings and flavours 1.4%

The ingrédient Equacia® available from CNI is a mixture of 90% gum acacia co-dried with 10% wheat cellulose.

Method:

1/ Add 10 wt. % water to the concentrated strawberry puree and heat to 85°C
2/ Prepare a solution of native guar and gum acacia :

- mix the powders
- disperse in 60 weight % water at 50°C under stirring

3/ Add this solution to the fruit puree and heat to 85°C
4/ Add sweeteners and colourings dispersed in the remaining water at 50°C
5/ Cool to 60°C
6/ Add flavouring and citric acid to adjust the pH to 4

A fairly fluid strawberry preparation can be obtained in this way.

This strawberry preparation without the use of specific stabilizers (e.g. Equacia® supplied by CNI) is not stable however when stored at 10°C. Macroscopic phase separation is observed: a liquid phase rich in gum acacia appears at the bottom of the pot after a few hours of storage at 10°C.

**Example 3: Preparation of fermented dairy products rich in guar gum:**

Fermented dairy products containing up to 2 weight % guar gum can be obtained by mixing the above-descried fruit preparations with a stirred yoghurt. The consistency of this mixture changes during the first minutes then becomes stable over time. The use of harmonic oscillation measurements allows the development of this mixture to be followed without mechanical disturbance. The principle is to subject a material to time sinusoid deformation, with a frequency f and a sufficiently low deformation level so that stress remains proportional to deformation. Under these conditions, the sample responds by exerting a response stress which is also a time sinusoid function, but with a phase shift. This shift indicates the balance between the solid contribution (or elastic, quantified by the elastic modulus G') and the liquid contribution (or viscous, quantified by the viscous modulus G" ) in the material response:

Complex viscosity η* is defined by:

$$\eta^* = \frac{\sqrt{G'^2 + G''^2}}{2 \cdot \pi \cdot f}$$

in which G' and G" are respectively the elastic and viscous moduli (expressed in Pa) and f is the frequency of the deformation (expressed in s-1). The chosen frequencies and deformations here are 1 Hz and 0.1%, which allows positioning within linear viscoelasticity. Measurement geometry is a mobile scissometer with six wings of diameter 2 cm marketed by AntonPaar - Physica (FL100). The scissometer is dipped into the centre of the sample and limits de-structuring thereof, owing to its small contact surface in the horizontal plane. Measurement is taken at 10 $\pm$ 0.1°C, the sample being inserted in a measurement chamber thermostat-regulated by Peltier effect (TEZ 150 PC).

The method used allows follow-up of the changes undergone by the mixture of white mass and fruit preparation, under conditions of such low mechanical disturbance that the mixture can be considered to be at rest.

Figure 8 shows changes over time of the complex viscosity of mixtures consisting of 80% hyper-protein white mass (containing 6.5% proteins) and 20% fruit preparation A (containing 7.5% guar gum and 12.5% gum acacia) or fruit preparation B (containing 7.5% guar gum and 20% gum acacia). Finally, product A contains 1.5% guar gum and 2.5% gum acacia, and product B contains 1.5% guar gum and 4% gum acacia.

In figure 8, the mixture prepared with fruit preparation A is shown as a continuous line and the preparation with fruit preparation B by symbols.

An increase in consistency is found right from the first minutes. It becomes weaker after around 30 minutes, then stabilizes around a plateau value after several hours. The following table gives a few complex viscosity values at different times and also gives the rate of increase in complex viscosity between two times under consideration, calculated as follows:

$$Rate = \frac{Vis\cos ity\,(t2) - vis\cos ity\,(t1)}{(t2 - t1)}$$

| Time | Complex viscosity of the mixture with fruit preparation A (in Pa.s) | Rate of increase in complex viscosity with preparation A (in Pa) | Complex viscosity of the mixture with fruit preparation B (in Pa.s) | Rate of increase in complex viscosity with preparation B (in Pa) |
|---|---|---|---|---|
| 1 minute | 178 | | 185 | |
| 10 minutes | 211 | 3.646 | 229 | 4.933 |
| 30 minutes | 221 | 0.336 | 245 | 0.763 |
| 60 minutes | 224 | 0.057 | 252 | 0.236 |
| 2 hours | 227 | 0.024 | 259 | 0.118 |
| 24 hours | 245 | 0.012 | 291 | 0.024 |
| 68 hours | 259 | 0.003 | 309 | 0.007 |

The table clearly shows that complex viscosity essentially changes over a short time and that a plateau is reached 24h after mixing at 10°C.

It is also observed that complex viscosity increases more when the fruit preparation contains more gum acacia: the difference is relatively limited (around 30 Pa) but is systematic. The ratio of guar gum and gum acacia used is therefore a lever to control the texture level of the end product.

Globally, the increased consistency observed could derive from osmotic swelling of the two polymers (guar and acacia gums) during mixing with the white mass, which contains 85% water. During digestion, the product is again diluted, both by fluids ingested during food intake (e.g. drinking water) and by digestive fluids (saliva, gastric juices, etc...). This dilution in turn allows osmotic swelling of the two polymers until they individually become fully hydrated and a homogeneous phase is obtained. This swelling is associated with an increase in viscosity which partly offsets the drop in viscosity with dilution, thereby giving end products whose viscosity is little affected by dilution, as shown in example 4.

Another advantage of the invention is that the high viscosity generated by the high dose of guar in the end product is also maintained during digestion.

To validate this result, an *in vitro* test to evaluate the viscosity generated by the fibres during digestion was developed. This test comprises a gastric phase followed by an intestinal phase.

The steps are the following:

1) the end product containing the fibres is first heated to 37°C for 30 minutes (step 1),
2) then acidified to pH2 using a 4N HCl solution (step 2),
3) after 10 minutes of continual stirring, 1.25 weight % of Sigma P7000 pepsin is then added in powder form to hydrolyse the protein microgels of the yoghurt (step 3),
4) after waiting 35 minutes, corresponding to gastric half-emptying for a fluid product, the pH is raised to 6 using concentrated 4N sodium hydroxide (NaOH), which corresponds to the pH of the small intestine (step 4).
5) after stirring for 10 minutes, 0.5 weight % of powder pancreatin, Sigma P7545, is added (step 5). Pancreatin contains different enzymes, including proteases, lipases and amylases. Stirring is continued for 10 minutes.

At each of the identified steps, a sample is taken and its viscosity measured using a MCR300 rheometer from Physica-Anton Paar. A flow curve is plotted, with a ramp-up of between 1 and 100 s$^{-1}$ in 3 minutes, then a ramp-down between 100 and 1 s$^{-1}$ in 3 minutes, with geometric progression of shear rates.

These flow curves are analysed at two levels:

- qualitatively, the shape of the flow curve gives information on the ingredient or ingredients generating viscosity in the product: a typical result obtained is a highly rheofluidifying and thixotropic behaviour at step 1, attributable to reversible orientation (for the rheofluidifying nature) and to partly irreversible breakdown (for thixotropy) of the protein microgels. At step 5, a behaviour of rather more polymer solution type is observed, with a Newtonian plateau at low rate and a rheofluidifying nature at faster rate.
- quantitatively, the numerical value of viscosity at a rate of 10 s$^{-1}$, representing the shear undergone by the food bolus in the small intestine, is extracted from the flow curves.

As an example, figure 7 below shows an *in vitro* test result obtained with a low-fat, stirred yoghurt of Taillefine® Brassé Nature type, and the same yoghurt mixed with a fruit preparation containing 5 weight % native guar gum and 10 weight % gum acacia. The proportion of white mass and fruit preparation being respectively 80 and 20 weight % in the end product, the quantities of guar and acacia gum are respectively 1 weight % and 2 weight % in the end product.

- The viscosity of the 2 yoghurts remains relatively unchanged by acidification to pH2 (step 2).
- On the other hand, after adding pepsin (step 3), the stirred yoghurt shows a strong decrease in viscosity attributable to de-structuring of the protein microgels through the addition of protease at a high dose. For the yoghurt containing guar, the decrease is much smaller than in the standard yoghurt.
- The return to pH 6 (step 4) again contributes towards a marked reduction in the viscosity of the Taillefine® Brassé Nature yoghurt, which can be attributed to re-solubilisation of the proteins at a pH which has become higher than isolectric pH. As in step 3, a limited decrease in observed in the yoghurt containing the guar/acacia mixture.
- The addition of pancreatin (step 5) does not lead to any change in viscosity, the two products not containing any complex glucids or lipids, whose contribution to texture could have an impact at this step. This step illustrates the strong difference in viscosity in the intestine: apparent viscosity at 10 s$^{-1}$ increases from 4.7 10$^{-3}$ Pa.s for Taillefine® Brassé Nature to 1.6 Pa.s for the same white mass, mixed with a fruit preparation so as to provide 1 weight % native guar gum and 2 weight % gum acacia (in the end product).

The *in vitro* test therefore confirms that the invention ultimately allows yoghurts to be obtained which, during digestion, generate viscosities that are much higher (340 times in the above example) than a standard yoghurt.

**Example 4: Fruit puree with satiating benefit:**

Fruit purees with significant contents of guar gum can be prepared according to the invention. One exemplary formulation is as follows:

| | |
|---|---|
| Concentrated (x3) apple puree | 23% |
| Blackcurrant puree | 12% |
| Sugar | 4% |
| Gum acacia | 7.35% |
| Guar gum | 2.5% |
| Wheat fibre | 0.15% |
| Water | 51% |

NB: the wheat fibre is incorporated using the commercial product Equacia®, consisting of 90% gum acacia and 10% wheat fibre.

Method of production:

Step 1: mixing gum acacia, Equacia®, guar gum and water
Step 2: adding concentrated apple puree and blackcurrant puree
Step 3: cooking (90°C for 5 minutes)
Step 4: cooling and packaging

A fruit puree that is sufficiently fluid to be consumed by sucking from a pack can be obtained in this manner. The nutritional composition is compared with a commercial fruit puree below:

| | Satiating puree | ANDROS commercial apple/blackcurrant puree |
|---|---|---|
| Calories | 64 kCal | 82 kCal |
| Carbohydrates | 11.5 g/100g | 19 g/100g |
| Lipids | 0.22 g/100g | 0.5 g/100g |
| Proteins | 0.28 g/100g | 0.4 g/100g |
| Fibres | 8.8 g/100g (30% RDA) | Not mentioned on pack |

The viscosity of the products (at 10 s$^{-1}$, as previously) was measured on the starting product and on the product after dilution (100 g of product and 200 g added water):

| | Viscosity of end product (Pa.s) | Viscosity after dilution (Pa.s) | Loss of viscosity on dilution |
|---|---|---|---|
| Satiating puree | 6 | 0.9 | Factor 6.7 |
| ANDROS apple/ blackcurrant puree | 4 | 0.005 | Factor 800 |

The right column indicates the factor of loss of viscosity due to dilution: the satiating puree of the invention only loses factor 6.7 viscosity, whereas the commercial puree has a viscosity drop by factor 800.

It therefore appears that the ternary mixture of fibres according to the invention allows a viscosity decrease due to dilution to be highly reduced.

Digestion is also associated with food dilution: ingestion of water concomitantly with food intake, saliva, gastric juices, etc... Having regard to the specific behaviour of the fibre mixtures described in the invention, viscosity is more extensively maintained during digestion, which imparts a satiating nature to the food.

**Claims**

1. Stable, fluid or semi-fluid food product having a dry extract of less than 30 weight % compared with the total weight of the product, advantageously less than 20 weight %, containing between 1 and 24 weight % fibres relative to the total weight of the food product, **characterized in that** the fibres consist of a mixture of :

   A) 0.4 to 5 weight %, relative to the total weight of the product, of viscosifying hydrosoluble polysaccharide fibres.
   B) 0.8 to 20 weight %, relative to the total weight of the product, of non-viscosifying hydrosoluble fibres, having a mean molar mass of between $3.10^5$ and $3.10^6$ g/mol and an intrinsic viscosity in aqueous solution of less than 0.3 dl/g.
   C) 0.04 to 0.6 weight %, relative to the total weight of the product, of non-hydrosoluble cellulose fibres.

2. Food product according to claim 1, **characterized in that** the viscosifying hydrosoluble polysaccharide fibres are of natural, plant origin and advantageously chosen from among carouba gum, fenugreek, konjac glucomannan, tara gum, oat and barley beta-glucans, guar gum, pectin and fibres of orange pulp.

3. Food product according to claim 2, **characterized in that** the viscosifying hydrosoluble polysaccharide fibres are chosen from among guar gum, carouba gum, konjac glucomannan and oat or barley beta-glucans or their mixtures.

4. Food product according to any of the preceding claims, **characterized in that** the non-viscosifying hydrosoluble polysaccharide fibres are chosen from among gum acacia, hydrosoluble soy or apple fibres or their mixtures, advantageously from among gum acacia and hydrosoluble apple fibres.

5. Food product according to any of the preceding claims, **characterized in that** the non-hydrosoluble cellulose fibres are wheat, cotton or wood fibres or their mixtures, advantageously wheat fibres.

6. Food product according to any of the preceding claims **characterized in that** it has a viscosity of more than 0.2 Pa.s under digestion conditions.

7. Food product according to any of the preceding claims, **characterized in that** it is chosen from among fresh dairy products, plant juices, beverages and their mixtures, advantageously from among fresh dairy products, fruit and/or vegetable juices, flavoured waters and fruit purees.

8. Food product according to any of the preceding claims, **characterized in that** it is stable for at least 4 weeks at 4°C, advantageously for 12 months at ambient temperature.

9. Food product according to claim 7 or 8, **characterized in that** it is a fresh dairy product with fruit and that the weight content of each of the fibres, relative to the total weight of the product, is as follows:

   A1) 0.4 to 2 weight % viscosifying hydrosoluble polysaccharide fibres,
   B1) 0.8 to 8% non-viscosifying hydrosoluble polysaccharide fibres, and
   C1) 0.09 to 0.25% non-hydrosoluble cellulose fibres.

10. Fresh dairy product according to any of claims 7 to 9, **characterized in that** it is chosen from among yoghurts, drink yoghurts, fromages frais and fermented milks.

11. Fresh dairy product according to any of claims 7 to 10, **characterized in that** it is low in fat and sugar.

12. Fresh dairy product according to any of claims 7 to 11, **characterized in that** it contains between 2 and 10 weight % proteins relative to the total weight of the fresh dairy product, advantageously between 4 and 7 weight % of proteins.

13. Fresh dairy product according to any of claims 7 to 12, **characterized in that** it contains fruit.

14. Fruit puree according to claim 7 or 8 **characterized in that** it contains:

A2) between 1 and 5 weight %, relative to the total weight of the puree, of viscosifying hydrosoluble polysaccharide fibres, advantageously 2.5 weight %;

B2) between 2 and 20 weight %, relative to the total weight of the puree, of non-viscosifying hydrosoluble polysaccharide fibres, advantageously 7.4 weight %, and

C2) between 0.05 and 0.6 weight %, relative to the total weight of the puree, of non-hydrosoluble cellulose fibres, advantageously 0.15 weight %.

15. Method to produce a fluid or semi-fluid food product according to any of claims 1 to 14, comprising the following steps:

   a) adding fibres such as defined in any of claims 1 to 5, to an initial matrix, and
   b) mixing the product obtained.

16. Production method according to claim 15 comprising a step ($\alpha$), prior to step (a), to disperse non-hydrosoluble cellulose fibres in non-viscosifying hydrosoluble polysaccharide fibres, by:

   - $\alpha$1) co-drying the non-hydrosoluble cellulose fibres with the non-viscosifying hydrosoluble polysaccharide fibres, or
   - $\alpha$2) mixing under strong shear, advantageously of more $10^4$ s$^{-1}$, the non-hydrosoluble cellulose fibres with the non-viscosifying hydrosoluble polysaccharide fibres, or
   - $\alpha$3) homogenizing under pressure, advantageously of at least 50 bars, a mixture of non-hydrosoluble cellulose fibres and non-viscosifying hydrosoluble polysaccharide fibres.

17. Production method according to claim 15 or 16, **characterized in that** the fibres added at step (a) are in the form of an intermediate preparation, advantageously chosen from among fruit preparations and syrups.

18. Intermediate preparation, advantageously intended to be used in a fluid or semi-fluid food product, **characterized in that** it contains:

   A3) between 2 and 10 weight %, relative to the total weight of the intermediate preparation, of viscosifying hydrosoluble polysaccharide fibres,
   B3) between 4 and 40 weight %, relative to the total weight of the intermediate preparation, of non-viscosifying hydrosoluble polysaccharide fibres, and
   C3) between 0.2 and 1.25 weight %, relative to the total weight of the intermediate preparation, of non-hydrosoluble cellulose fibres.

19. Use of the intermediate preparation according to claim 18 in a fluid or semi-fluid food product, advantageously in a fresh dairy product.

20. Method to produce an intermediate preparation according to claim 18 comprising the following successive steps:

   - mixing the different fibres in powder form,
   - dispersing this mixture of powders in water under stirring,
   - optionally, adding fruit, sugar, colouring agents, flavourings to this dispersion,
   - pasteurising heat treatment of the preparation thus obtained,
   - cooling the intermediate preparation thus obtained,
   - storing the intermediate preparation in a low-temperature container.

21. Food product according to any of claims 1 to 14 for its use as medicinal product.

22. Food product according to claim 21 for its use as satiating food to increase the feeling of gastric fullness, to delay the onset of feelings of hunger and/or to manage a person's body weight and/or as functional food reducing an individual's circulating blood cholesterol and delaying glycaemic and insulinaemic responses of such individual after a meal, and thereby preventing the onset of symptoms of metabolic syndrome and/or as functional food reducing the level of an individual's circulating blood cholesterol and preventing the onset of symptoms related to cardiovascular disorders.

**Patentansprüche**

1. Stabiles, flüssiges oder halbflüssiges Nahrungsmittelprodukt mit einem Trockenextrakt von weniger als 30 Gew.-%, bevorzugt weniger als 20 Gew.-%, bezogen auf das Gesamtgewicht des Produkts, enthaltend zwischen 1 und 24 Gew.-% Fasern, bezogen auf das Gesamtgewicht des Nahrungsmittelprodukts, **dadurch gekennzeichnet, dass** die Fasern aus einer Mischung bestehen von:

   A) 0,4 bis 5 Gew.-%, bezogen auf das Gesamtgewicht des Produkts, viskosifizierenden wasserlöslichen Polysaccharidfasern;
   B) 0,8 bis 20 Gew.-%, bezogen auf das Gesamtgewicht des Produkts, nicht-viskosifizierenden wasserlöslichen Fasern mit einer mittleren molaren Masse zwischen $3 \cdot 10^5$ und $3 \cdot 10^6$ g/mol und einer Grenzviskosität in wässriger Lösung von weniger als 0,3 dl/g;
   C) 0,04 bis 0,6 Gew.-%, bezogen auf das Gesamtgewicht des Produkts, nicht-wasserlöslichen Zellulosefasern.

2. Nahrungsmittelprodukt gemäß Anspruch 1, **dadurch gekennzeichnet, dass** die viskosifizierenden wasserlöslichen Polysaccharidfasern natürlichen pflanzlichen Ursprungs sind und bevorzugt ausgewählt sind aus Caroubagummi, Bockshornklee, Konjac-Glucomannan, Tara-Gummi, Hafer- und Gersten-Beta-Glucanen, Guargummi, Pektin und Orangenpulpe-Fasern.

3. Nahrungsmittelprodukt gemäß Anspruch 2, **dadurch gekennzeichnet, dass** die viskosifizierenden wasserlöslichen Polysaccharidfasern ausgewählt sind aus Guargummi, Caroubagummi, Konjac-Glucomannan, und Hafer- oder Gersten-Beta-Glucanen oder deren Mischungen.

4. Nahrungsmittelprodukt gemäß irgendeinem der vorhergehenden Ansprüche, **dadurch gekennzeichnet, dass** die nicht-viskosifizierenden wasserlöslichen Polysaccharidfasern ausgewählt sind aus Acacia-Gummi, wasserlöslichen Soja- oder Apfelfasern oder deren Mischungen, bevorzugt aus Acacia-Gummi und wasserlöslichen Apfelfasern.

5. Nahrungsmittelprodukt gemäß irgendeinem der vorhergehenden Ansprüche, **dadurch gekennzeichnet, dass** es sich bei den nicht-wasserlöslichen Zellulosefasern um Weizen-, Baumwoll- oder Holzfasern oder deren Mischungen , bevorzugt Weizenfasern, handelt.

6. Nahrungsmittelprodukt gemäß irgendeinem der vorhergehenden Ansprüche, **dadurch gekennzeichnet, dass** es eine Viskosität von mehr als 0,2 Pa.s unter Verdauungsbedingungen aufweist.

7. Nahrungsmittelprodukt gemäß irgendeinem der vorhergehenden Ansprüche, **dadurch gekennzeichnet, dass** es ausgewählt ist aus Frischmilchprodukten, Pflanzensäften, Getränken und deren Mischungen, bevorzugt aus Frischmilchprodukten, Frucht- und/oder Gemüsesäften, aromatisierten Wässern und Fruchtpürees.

8. Nahrungsmittelprodukt gemäß irgendeinem der vorhergehenden Ansprüche, **dadurch gekennzeichnet, dass** es für mindestens 4 Wochen bei 4 °C, bevorzugt für 12 Monate bei Umgebungstemperatur, stabil ist.

9. Nahrungsmittelprodukt gemäß Anspruch 7 oder 8, **dadurch gekennzeichnet, dass** es sich um ein Frischmilchprodukt mit Frucht handelt, und dass der Gewichtsgehalt jeder der Fasern, bezogen auf das Gesamtgewicht des Produkts, folgendermaßen ist:

   A1) 0,4 bis 2 Gew.-% viskosifizierende wasserlösliche Polysaccharidfasern;
   B1) 0,8 bis 8 % nicht-viskosifizierende wasserlösliche Polysaccharidfasern;
   C1) 0,04 bis 0,25 % nicht-wasserlösliche Zellulosefasern.

10. Frischmilchprodukt gemäß irgendeinem der Ansprüche 7 bis 9,
    **dadurch gekennzeichnet, dass** es ausgewählt ist aus Joghurts, Trinkjoghurts, Frischkäsen und fermentierten Milchprodukten.

11. Frischmilchprodukt gemäß irgendeinem der Ansprüche 7 bis 10, **dadurch gekennzeichnet, dass** es einen niedrigen Fett- und Zuckergehalt aufweist.

12. Frischmilchprodukt gemäß irgendeinem der Ansprüche 7 bis 11, **dadurch gekennzeichnet, dass** es zwischen 2 und 10 Gew.-% Proteine, bevorzugt zwischen 4 und 7 Gew.-% Proteine, bezogen auf das Gesamtgewicht des Frischmilchprodukts, enthält.

**13.** Frischmilchprodukt gemäß irgendeinem der Ansprüche 7 bis 12, **dadurch gekennzeichnet, dass** es Frucht enthält.

**14.** Fruchtpüree gemäß Anspruch 7 oder 8, **dadurch gekennzeichnet, dass** es enthält:

A2) zwischen 1 und 5 Gew.-%, bevorzugt 2,5 Gew.-%, bezogen auf das Gesamtgewicht des Pürees, viskosifizierende wasserlösliche Polysaccharidfasern;
B2) zwischen 2 und 20 Gew.-%, bevorzugt 7,4 Gew.-%, bezogen auf das Gesamtgewicht des Pürees, nicht-viskosifizierende wasserlösliche Polysaccharidfasern; und
C2) zwischen 0,05 und 0,6 Gew.-%, bevorzugt 0,15 Gew.-%, bezogen auf das Gesamtgewicht des Pürees, nicht-wasserlösliche Zellulosefasern.

**15.** Verfahren zur Herstellung eines flüssigen oder halbflüssigen Nahrungsmittelprodukts gemäß irgendeinem der Ansprüche 1 bis 14, umfassend die folgenden Schritte:

a) Zugabe von Fasern, wie in irgendeinem der Ansprüche 1 bis 5 definiert, zu einer Ausgangsmasse, und
b) Mischen des erhaltenen Produkts.

**16.** Herstellungsverfahren gemäß Anspruch 15, umfassend einen Schritt ($\alpha$) vor dem Schritt (a) zum Dispergieren der nicht-wasserlöslichen Zellulosefasern in den nicht-viskosifizierenden wasserlöslichen Polysaccharidfasern durch:

- $\alpha$1) gemeinsames Trocknen der nicht-wasserlöslichen Zellulosefasern mit den nicht-viskosifizierenden wasserlöslichen Polysaccharidfasern, oder
- $\alpha$2) Mischen bei starker Scherung, bevorzugt von mehr als $10^4$ s$^{-1}$, der nicht-wasserlöslichen Zellulosefasern mit den nicht-viskosifizierenden wasserlöslichen Polysaccharidfasern, oder
- $\alpha$3) Homogenisieren unter Druck, bevorzugt von wenigstens 50 Bar, einer Mischung der nicht-wasserlöslichen Zellulosefasern und nicht-viskosifizierenden wasserlöslichen Polysaccharidfasern.

**17.** Herstellungsverfahren gemäß Anspruch 15 oder 16, **dadurch gekennzeichnet, dass** die in Schritt (a) zugegebenen Fasern in Form einer Zwischenaufbereitung, bevorzugt ausgewählt aus Fruchtaufbereitungen und Sirupen, vorliegt.

**18.** Zwischenaufbereitung, bevorzugt zur Verwendung in einem flüssigen oder halbflüssigen Nahrungsmittelprodukt, **dadurch gekennzeichnet, dass** sie enthält:

A3) zwischen 2 und 10 Gew.-%, bezogen auf das Gesamtgewicht der Zwischenaufbereitung, viskosifizierende wasserlösliche Polysaccharidfasern;
B3) zwischen 4 und 40 Gew.-%, bezogen auf das Gesamtgewicht der Zwischenaufbereitung, nicht-viskosifizierende wasserlösliche Polysaccharidfasern; und
C3) zwischen 0,2 und 1,25 Gew.-%, bezogen auf das Gesamtgewicht der Zwischenaufbereitung, nicht-wasserlösliche Zellulosefasern.

**19.** Verwendung der Zwischenaufbereitung gemäß Anspruch 18 in einem flüssigen oder halbflüssigen Nahrungsmittelprodukt, bevorzugt in einem Frischmilchprodukt.

**20.** Verfahren zur Herstellung einer Zwischenaufbereitung gemäß Anspruch 18, umfassend die folgenden aufeinander folgenden Schritte:

- Mischen der verschiedenen Fasern in Pulverform,
- Dispergieren dieser Pulvermischung in Wasser unter Rühren,
- optional, Zugabe von Frucht, Zucker, Farbstoffen, Aromen zu dieser Suspension,
- Pasteurisierende Wärmebehandlung der so erhaltenen Aufbereitung,
- Abkühlen der so erhaltenen Zwischenaufbereitung,
- Lagern der Zwischenaufbereitung in einem Behälter bei niedriger Temperatur.

**21.** Nahrungsmittelprodukt gemäß irgendeinem der Ansprüche 1 bis 14 zur Verwendung als medizinisches Produkt.

**22.** Nahrungsmittelprodukt gemäß Anspruch 21 zur Verwendung als Sättigungsnahrungsmittel zur Erhöhung des Gefühls der Magenvölle, zur Verzögerung des Einsetzens des Hungergefühls und/oder zur Kontrolle des Körpergewichts eines Individuums und/oder als funktionelles Nahrungsmittel, welches das zirkulierende Blutcholesterin eines Individuums reduziert und glykämische und insulinämische Antworten eines derartigen Individuums nach einer Mahlzeit verzögert und dadurch das Einsetzen von Symptomen des metabolischen Syndroms verhindert, und/oder als funktionelles Nahrungsmittel, welches das Niveau des zirkulierenden Blutcholesterins eines Individuums reduziert und das Einsetzen von Symptomen in Verbindung mit kardiovaskulären Erkrankungen verhindert.

**Revendications**

1. Produit alimentaire fluide ou semi-fluide stable ayant un extrait sec inférieur à 30 % en poids par rapport au poids total du produit, avantageusement inférieur à 20 % en poids, comprenant entre 1 et 24 % en poids de fibres par rapport au poids total du produit alimentaire, **caractérisé en ce que** les fibres sont constituées par un mélange :

   A) de 0,4 à 5 % en poids, par rapport au poids total du produit, de fibres polysaccharidiques hydrosolubles viscosifiantes
   B) de 0,8 à 20 % en poids, par rapport au poids total du produit, de fibres hydrosolubles non viscosifiantes ayant une masse molaire moyenne comprise entre $3.10^5$ et $3.10^6$ g/mol et une viscosité intrinsèque en solution aqueuse inférieure à 0,3 dl/g
   C) de 0,04 à 0,6 % en poids, par rapport au poids total du produit, de fibres cellulosiques non hydrosolubles.

2. Produit alimentaire selon la revendication 1, **caractérisé en ce que** les fibres polysaccharidiques hydrosolubles viscosifiantes sont d'origine végétale naturelle et avantageusement choisies parmi la gomme de caroube, le fenugrec, le glucomannane de konjac, la gomme tara, les beta-glucanes d'avoine et d'orge, la gomme de guar, la pectine et les fibres de pulpe d'orange.

3. Produit alimentaire selon la revendication 2, **caractérisé en ce que** les fibres polysaccharidiques hydrosolubles viscosifiantes sont choisies parmi la gomme de guar, la gomme de caroube, le glucomannane de konjac et les beta-glucanes d'avoine ou d'orge ou leurs mélanges.

4. Produit alimentaire selon l'une quelconque des revendications précédentes, **caractérisé en ce que** les fibres polysaccharidiques hydrosolubles non viscosifiantes sont choisies parmi la gomme d'acacia, les fibres de pomme ou de soja hydrosolubles ou leurs mélanges, avantageusement parmi la gomme d'acacia et les fibres de pomme hydrosolubles.

5. Produit alimentaire selon l'une quelconque des revendications précédentes, **caractérisé en ce que** les fibres cellulosiques non hydrosolubles sont les fibres de blé, de coton ou de bois ou leurs mélanges, avantageusement les fibres de blé.

6. Produit alimentaire selon l'une quelconque des revendications précédentes, **caractérisé en ce qu'**il a une viscosité supérieure à 0,2 Pa.s en condition de digestion.

7. Produit alimentaire selon l'une quelconque des revendications précédentes, **caractérisé en ce qu'**il est choisi parmi les produits laitiers frais, les jus végétaux, les boissons et leurs mélanges, avantageusement parmi les produits laitiers frais, les jus de fruits et/ou de légumes, les eaux aromatisées ou les purées de fruits.

8. Produit alimentaire selon l'une quelconque des revendications précédentes, **caractérisé en ce qu'**il est stable pendant au moins 4 semaines à 4°C, avantageusement pendant 12 mois à température ambiante.

9. Produit alimentaire selon la revendication 7 ou 8, **caractérisé en ce qu'**il s'agit d'un produit laitier frais aux fruits et que la teneur en poids, par rapport au poids total du produit, en chacune des fibres est la suivante :

   A1) 0,4 à 2 % de fibres polysaccharidiques hydrosolubles viscosifiantes,
   B1) 0,8 à 8 % de fibres polysaccharidiques hydrosolubles non viscosifiantes, et
   C1) 0,04 à 0,25 % de fibres cellulosiques non hydrosolubles.

10. Produit laitier frais selon l'une quelconque des revendications 7 à 9, **caractérisé en ce qu'**il est choisi parmi les yaourts, les yaourts à boire, les fromages frais et les laits fermentés.

**11.** Produit laitier frais selon l'une quelconque des revendications 7 à 10, **caractérisé en ce qu'**il est pauvre en matières grasses et en sucre.

**12.** Produit laitier frais selon l'une quelconque des revendications 7 à 11, **caractérisé en ce qu'**il contient entre 2 et 10 % en poids de protéines par rapport au poids total du produit laitier frais, avantageusement entre 4 et 7 % en poids de protéines.

**13.** Produit laitier frais selon l'une quelconque des revendications 7 à 12, **caractérisé en ce qu'**il contient des fruits.

**14.** Purée de fruits selon la revendication 7 ou 8 **caractérisée en ce qu'**elle contient :

A2) entre 1 et 5 % en poids, par rapport au poids total de la purée, de fibres polysaccharidiques hydrosolubles viscosifiantes, avantageusement 2,5 % en poids ;
B2) entre 2 et 20 % en poids, par rapport au poids total de la purée, de fibres polysaccharidiques hydrosolubles non viscosifiantes, avantageusement 7,4 % en poids et
C2) entre 0,05 et 0,6 % en poids, par rapport au poids total de la purée, de fibres cellulosiques non hydrosolubles, avantageusement 0,15 % en poids.

**15.** Procédé de fabrication d'un produit alimentaire fluide ou semi-fluide, selon l'une quelconque des revendications 1 à 14, comprenant les étapes suivantes :

a) ajout des fibres telles que définies dans l'une quelconque des revendications 1 à 5, à une matrice initiale, et
b) mélange du produit obtenu.

**16.** Procédé de fabrication, selon la revendication 15, comprenant une étape ($\alpha$), préalable à l'étape (a), de dispersion des fibres cellulosiques non hydrosolubles dans les fibres polysaccharidiques hydrosolubles non viscosifiantes, par :

- $\alpha$1) co-séchage des fibres cellulosiques non hydrosolubles avec les fibres polysaccharidiques hydrosolubles non viscosifiantes, ou
- $\alpha$2) mélange sous fort cisaillement, avantageusement supérieur à $10^4$ s$^{-1}$, des fibres cellulosiques non hydrosolubles avec les fibres polysaccharidiques hydrosolubles non viscosifiantes, ou
- $\alpha$3) homogénéisation sous pression, avantageusement d'au moins 50 bars, d'un mélange des fibres cellulosiques non hydrosolubles et des fibres polysaccharidiques hydrosolubles non viscosifiantes.

**17.** Procédé de fabrication, selon la revendication 15 ou 16, **caractérisé en ce que** les fibres ajoutées à l'étape (a) se trouvent sous la forme d'une préparation intermédiaire, avantageusement choisie parmi les préparations de fruits et les sirops.

**18.** Préparation intermédiaire, avantageusement destinée à être utilisée dans un produit alimentaire fluide ou semi fluide, **caractérisée en ce qu'**elle contient :

A3) entre 2 et 10 % en poids, par rapport au poids total de la préparation intermédiaire, de fibres polysaccharidiques hydrosolubles viscosifiantes,
B3) entre 4 et 40 % en poids, par rapport au poids total de la préparation intermédiaire, de fibres polysaccharidiques hydrosolubles non viscosifiantes, et
C3) entre 0,2 et 1,25 % en poids, par rapport au poids total de la préparation intermédiaire, de fibres cellulosiques non hydrosolubles.

**19.** Utilisation de la préparation intermédiaire selon la revendication 18 dans un produit alimentaire fluide ou semi fluide, avantageusement dans un produit laitier frais.

**20.** Procédé de fabrication d'une préparation intermédiaire selon la revendication 18 comprenant les étapes successives suivantes :

- mélange des différentes fibres sous forme de poudres
- dispersion de ce mélange de poudres dans de l'eau sous agitation
- optionnellement, ajout à cette dispersion de fruits, de sucres, de colorants, d'arômes
- traitement thermique de pasteurisation de la préparation ainsi obtenue

- refroidissement de la préparation intermédiaire ainsi obtenue
- stockage de la préparation intermédiaire dans un container à basse température.

21. Produit alimentaire selon l'une quelconque des revendications 1 à 14, pour son utilisation comme produit médical.

22. Produit alimentaire selon la revendication 21, pour son utilisation en tant qu'aliment satiétogène pour augmenter la sensation de plénitude gastrique, retarder l'apparition de la sensation de faim et/ou pour gérer le poids corporel d'un individu et/ou en tant qu'aliment fonctionnel diminuant le cholestérol dans la circulation sanguine d'un individu et ralentissant les réponses glycémiques et insulinémiques de cet individu à la suite d'un repas de sorte à prévenir l'apparition des symptômes du syndrome métabolique et/ou en tant qu'aliment fonctionnel diminuant le taux de cholestérol dans la circulation sanguine d'un individu et prévenant l'apparition de symptômes liés aux troubles cardiovasculaires.

## FIGURE 1

Concentration of Meyproguar 225 guar (%)

## FIGURE 2

● Meyproguar M225
◇ Meyproguar M225 & 10% Fibregum B
■ Meyproguar M225 & 12,5% Fibregum B
○ Meyproguar M225 & 20% Fibregum B

Concentration of Meyproguar 225 guar (%)

## FIGURE 3

## FIGURE 4

FIGURE 5

FIGURE 6

## FIGURE 7

Viscosity at 10 s-1 (Pa.s) and 37°C

pH2   pH6

End product, stirred
Taillefine containing
1% guar gum and
2% gum acacia

White mass of plain
Taillefine stirred
yoghurt (no fibres)

Step 1 : product at 37°C
Step 2: acidification to pH2
Step 3 : addition of pepsin
Step 4 : raising to pH6
Step 5 : addition of pancreatin

DIGESTION STEP

## FIGURE 8

Complex viscosity (Pa.s)

— Fresh product with preparation A
o Fresh product with preparation B

Time (minutes)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0067592 A
- US 2003013679 A
- WO 2005036971 A
- EP 1008306 A
- US 5545411 A

- JP 2005185132 B
- US 4988530 A
- US 4971810 A
- WO 2006134157 A
- FR 881203

**Non-patent literature cited in the description**

- **JASIM AHMED et al.** *Int. Journal of Food Properties,* 2005, vol. 8, 179-192
- **DOUBLIER ; WOOD.** *Cereal Chemistry,* 1995, vol. 72, 335-340
- **AL-ASSAF et al.** *Food Hydrocolloids,* 2005, vol. 19, 647-667

- **FLINDT et al.** *Food Hydrocolloids,* 2005, vol. 19, 687-701
- *Journal Officiel de la République Française,* 31 December 1988